# EUROPÄISCHE PATENTANMELDUNG

(11) **EP 2 181 656 A1**
(43) Veröffentlichungstag der Anmeldung: **05.05.2010**
(21) Anmeldenummer: 09174333.6
(22) Anmeldetag: 28.10.2009
(51) Int. Cl.: A61B 17/34, A61N 1/05

(54) **Vorrichtung zum Einbringen von medizinischen Implantaten**

(30) Priorität: 04.11.2008 DE 102008043452
(71) Anmelder: Biotronik CRM Patent AG, 6341 Baar (CH)
(72) Erfinder: Flach, Erhard, 12305, Berlin (DE)
(74) Vertreter: Lindner-Vogt, Karin L.

(57) **Zusammenfassung**

Gezeigt wird eine Vorrichtung (1) zum zielgerichteten dauerhaften oder vorübergehenden Einbringen von medizinischen Implantaten (2, 3) in ein Lebewesen, welche einen lang gestreckten Körper mit einem distalen Ende und einem proximalen Ende umfasst. Dabei ist die Vorrichtung so ausgelegt, dass es über einen Zugang in das Lebewesen geführt werden kann, so dass das proximale Ende außerhalb des Lebewesens verbleibt und dass das distale Ende eine Fixierungseinrichtung (11) aufweist, mit der die Vorrichtung (1) lösbar mit dem Körpergewebe (101) fest verbunden werden kann. Über die so fixierte Vorrichtung kann dann ein medizinisches Implantat (2, 3) gezielt im Gewebe (101) fixiert werden. Das Implantat zum dauerhaften oder vorübergehenden Verbleib in einem Lebewesen umfasst dabei einen durchgängig hohlen Hohlkörper (20, 30) entlang einer Längsachse mit einem distalen und einem proximalen Ende, wobei der Hohlkörper (20, 30) so beschaffen ist, dass er die erfindungsgemäße Vorrichtung (1) aufnehmen und reversibel führen kann, und jeweils einer Öffnung am proximalen und am distalen Ende, durch die die Vorrichtung dringen kann, wobei die Öffnung am distalen Ende eine von der Fixierungseinrichtung (11) der Vorrichtung reversibel durchstoßbare Dichteinheit (23) zur Abdichtung des durchgängig hohlen Hohlkörpers (20,30) aufweist.

## Beschreibung

Die Erfindung betrifft eine Vorrichtung zum zielgerichteten dauerhaften oder vorübergehenden Einbringen von medizinischen Implantaten in ein Lebewesen, welche einen lang gestreckten Körper mit einem distalen Ende und einem proximalen Ende umfasst, wobei die Vorrichtung ausgelegt ist, über einen Zugang in das Lebewesen geführt zu werden, so dass das proximale Ende außerhalb des Lebewesens verbleibt.

Weiterhin betrifft die Erfindung ein medizinisches Implantat zum dauerhaften oder vorübergehenden Verbleib in einem Lebewesen, das einen Hohlkörper mit einem distalen und einem proximalen Ende und mit einem durchgängigen Hohlraum entlang der Längsachse des Hohlkörpers umfasst, wobei der Hohlkörper so beschaffen ist, dass er die erfindungsgemäße Vorrichtung aufnehmen und reversibel führen kann, und weiterhin jeweils einer Öffnung am proximalen und am distalen Ende umfasst, durch die die Vorrichtung dringen kann.

Solche bekannten Vorrichtungen werden eingesetzt, um Elektrodenleitungen an einen gewünschten Implantationsort in ein Lebewesen zu verbringen, wo sie dann befestigt werden. Dabei wird unter anderem zuerst ein so genannter Führungsdraht durch das venöse System in eine Herzkammer oder ein Herzkranzgefäß gelegt, entlang diesem dann eine Elektrodenleitung eingeführt und am Körpergewebe wie der Herzwand fixiert wird. Problematisch wird es dann, wenn besondere Gegenden gezielt stimuliert werden sollen. Dies ist beispielsweise der Fall, wenn der Arzt Elektrodenleitungen hochseptal in der nähe des HIS-Bündels fixieren möchte. Dort ergibt sich das Problem, dass die Elektrodenleitung in der Regel mit einem Führungskatheter an den Ort der Fixierung gebracht werden muss. Auf Grund der engen Platzverhältnisse ist es schwierig, die Elektrodenleitung durch kleine Biegeradien des Führungskatheters in das Zielgebiet zu führen. Die zunehmende Reibung zwischen der Elektrodenleitung und Führungskatheter verhindert dann ein Vorschieben. Darüber hinaus kann der Arzt die Elektrodenleitung nicht mehr ausreichend an die Herzwand andrücken, da sie in einer 90°-Stellung zur Einführachse steht und der Katheter nicht genügend Stützmöglichkeiten bietet. Deshalb ist es sehr schwierig oder oft genug unmöglich, Standard-Elektroden wie aktiv fixierbare Schraubelektroden in Bereichen des Herzens zu befestigen, in denen die Elektroden nur noch tangential oder in einem flachen Winkel auf das Herzgewebe treffen. Ein Katheter der ausreichend große Biegeradien bietet, kann aber die Elektroden nur noch in einem flachen Winkel in das Zielgebiet führen, was ein Einschrauben auf Grund des mangelnden Andrucks ebenfalls verhindert.

Ebenso möglich ist es, von außen eine Elektrodenleitung mit einer ähnlichen Methode am Epikard zu befestigen. Dazu wird ein Führungskatheter durch die Brustwand eingebracht, in den dann eine Elektrodenleitung mit Fixierung geführt und an der äußeren Herzwand (Epikard) angebracht wird. Um an die Herzwand von außen heranzukommen, muss der relativ enge Raum zwischen Brustwand mit Rippen passiert werden. Deshalb kann die Elektrodenleitung dort nicht im rechten Winkel eingebracht werden, sondern es muss versucht werden, die Elektrode tangential an die Herzwand heranzuführen und dann zu befestigen. Eine handelsübliche Einschraubelektrodenleitung kann dann nicht mehr befestigt werden.

Es ist daher Aufgabe der vorliegenden Erfindung, eine Vorrichtung und damit zusammenwirkendes medizinisches Implantat zu schaffen, das sicherstellt, dass ein möglichst handelsübliches Implantat sicher und zielgerichtet an einem Körpergewebe so befestigt werden kann, dass der Anwender die Befestigung während des Einbringens und Befestigens der Elektrode gewährleisten kann.

Diese Aufgabe wird durch die Vorrichtung aus Anspruch 1 und das medizinische Implantat aus Anspruch 8 gelöst.

Der erste Aspekt der Aufgabe wird durch eine Vorrichtung zum zielgerichteten dauerhaften oder vorübergehenden Einbringen von medizinischen Implantaten in ein Lebewesen gelöst, welche einen lang gestreckten Körper mit einem distalen Ende und einem proximalen Ende umfasst. Dabei ist die Vorrichtung so ausgelegt, dass es über einen Zugang in das Lebewesen geführt werden kann, so dass das proximale Ende außerhalb des Lebewesens verbleibt und dass das distale Ende eine Fixierungseinrichtung aufweist, mit der die Vorrichtung lösbar mit dem Körpergewebe fest verbunden werden kann. Vorzugsweise ist die Fixierungseinrichtung dafür ausgebildet, in das Körpergewebe einzudringen, also vorzugsweise angespitzt und besonders bevorzugt nadelförmig. Damit ist der distale Bereich der Vorrichtung am Myokard temporär fixiert, indem die Fixiervorrichtung im Körpergewebe eingedrungen ist. Über die so fixierte Vorrichtung kann dann ein medizinisches Implantat gezielt im Gewebe fixiert werden. Das Implantat zum dauerhaften oder vorübergehenden Verbleib in einem Lebewesen umfasst dabei einen durchgängig hohlen Hohlkörper entlang einer Längsachse mit einem distalen und einem proximales Ende, wobei der Hohlkörper so beschaffen ist, dass er die erfindungsgemäße Vorrichtung aufnehmen und reversibel führen kann, und jeweils einer Öffnung am proximalen und am distalen Ende, durch die die Vorrichtung dringen kann, wobei die Öffnung am distalen Ende eine von der Fixierungseinrichtung der Vorrichtung reversibel durchstoßbare Dichteinheit zur Abdichtung des durchgängig hohlen Hohlkörpers aufweist.

Gemäß einer weiteren bevorzugten Ausgestaltung weist die Fixierungseinrichtung eine in laterale Richtung erstreckende Vorbiegung auf, welche vorzugsweise einen Radius von 5 mm bis 20 mm und/oder einen Kreisbogen mit einem Winkel von 70° bis 90° im Winkelmaß aufweist. Dadurch kann das Implantat über die Vorrichtung zum Implantationsort gebracht werden, indem es beispielsweise im engen Raum zwischen Brustkorb und Perikard geführt wird, dann im besonders engen Raum im Perikard tangential raumsparend an das Epikard geführt und anschließend mit einem angemessenen Winkel auf das Myokard befestigen wird. So lassen sich die notwendigen Penetrationskräfte auf das Implantat aufbringen, ohne dass sie ausweichen kann.

Um die Herstellung zu erleichtern und herkömmliche Implantate verwenden zu können, weißt die Vorrichtung herkömmliche Eigenschaften auf, wie beispielsweise, dass der Körper und die Fixierungseinrichtung elastisch sind, vorzugsweise bestehend aus einem Draht, besonders bevorzugt aus einem Draht mit einem Durchmesser von kleiner gleich 0,36 mm.

Um beim Einführen der Vorrichtung Verletzungen zu vermeiden weist die Vorrichtung vorzugsweise am proximalen Ende eine Arretierungsvorrichtung auf, welche eine lösbare Arretierung mittel- oder unmittelbar mit dem medizinischen Implantat ermöglicht. Dadurch wird die Lage zwischen Vorrichtung und medizinischen Implantat eingestellt oder eine Verschiebung zwischen Vorrichtung und Implantat verhindert.

Gemäß einer zusätzlichen Form umfasst die Vorrichtung weiter eine Führung zur reversiblen Führung oder Halterung der Vorrichtung, welche einen lang gestreckten Hohlkörper mit einem distalen und einem proximalen Ende mit einem durchgängigen Hohlraum entlang einer Längsachse aufweist, wobei der Hohlkörper so beschaffen ist, dass er die Vorrichtung aufnehmen und reversibel führen und/oder haltern kann. Weiter umfasst die Führung jeweils eine Öffnung am proximalen und am distalen Ende, welche mit dem Hohlraum des Hohlkörpers verbunden sind und durch die die Vorrichtung dringen kann, und eine Arretierungseinheit am proximalen Ende, welche eine lösbare Arretierung mittel- oder unmittelbar mit der Vorrichtung ermöglicht, um die Lage zwischen Vorrichtung und Führung einzustellen oder eine Verschiebung zwischen Vorrichtung und Führung zu verhindern. Vorzugsweise ist die Führung als Hülse, vorzugsweise als elastische biegbare Mandrinhülse ausgebildet. Dadurch kann das Einführen eines Implantates weiter verbessert werden, da dann die Vorrichtung gestützt und wesentlich stabiler ist. Weiterhin kann durch die zusätzliche Führung das Einführen und Platzieren der Elektrodenleitung wesentlich vereinfacht werden.

Der zweite Aspekt der Aufgabe wird durch ein medizinisches Implantat zum dauerhaften oder vorübergehenden Verbleib in einem Lebewesen gelöst welches einen Hohlkörper mit einem distalen und einem proximalen Ende und einem durchgängigen Hohlraum entlang einer Längsachse gelöst. Der Hohlkörper ist dabei so beschaffen, dass er die Vorrichtung zum zielgerichteten dauerhaften oder vorübergehenden Einbringen von medizinischen Implantaten in ein Lebewesen aufnehmen und reversibel führen kann. Der Hohlraum weist jeweils eine mit ihm verbundene Öffnung am proximalen und am distalen Ende auf, durch die die Vorrichtung dringen kann. Die Öffnung am distalen Ende weist dabei eine von der Fixierungseinrichtung der Vorrichtung zum zielgerichteten dauerhaften oder vorübergehenden Einbringen von medizinischen Implantaten in ein Lebewesen reversibel durchstoßbare Dichteinheit zur Abdichtung des Hohlkörpers auf.

Vorteilig ist an dem medizinischen Implantat, dass es so wenig wie möglich Anpassungen erfährt, um mit der erfindungsgemäßen Vorrichtung eingebracht werden zu können. So weist das Implantat vorzugsweise eine sich in seinem Hohlkörper unmittelbar proximal vor der Dichteinheit befindliche Anschlageinrichtung mit einer Bohrung und einem Widerlager auf, welche so ausgestaltet ist, dass die Fixierungseinrichtung der Vorrichtung die Bohrung durchdringen kann und die Führung am Widerlager anschlägt. Das ermöglicht das Führen des Implantats mit der Führung bzw. mit der Mandrinhülse, welche bei einer Kraftauswirkung entlang der Längsachse in distale Richtung am Widerlager anschlägt. Bei weiterer Kraftaufbringung wird das Implantat, welches durchaus aus sehr biegeweichem oder dünnem Material besteht, in distale Richtung getrieben, bis es am Implantationsort angelangt und dort fixiert werden kann.

Bevorzugt handelt es sich bei dem medizinischen Implantat um eine medizinische Elektrodenleitung, welche am distalen Ende mindestens einen elektrischen Pol zur Abgabe elektrischer Pulse auf das Körpergewebe des Lebewesens, mindestens einen mit jeweils einem dieser Pole elektrisch verbundenen Leiter, der sich vom jeweiligen Pol zum proximalen Ende erstreckt, und einer Anschlusseinheit am proximalen Ende, welche mit jedem der elektrischen Leiter verbunden ist, um eine elektrische Verbindung mit einem implantierbaren oder externen elektrischen Gerät herzustellen, umfasst. Solche Elektrodenleitungen werden in der modernen Medizin in vielfältiger Form eingesetzt. Sie können beispielsweise wie beschrieben in oder an einem Herzen angewandt werden, um die sichere Funktion des Herzens unmittelbar sicherstellen zu können. Weiterhin sind Elektrodenleitungen umfasst, die auch im Nervensystem oder in einem Gehirn eingesetzt werden. Diese Leitungen müssen extrem filigran sein, weshalb beispielsweise die Führung entlang einer Mandrinhülse von herausragender Wichtigkeit ist.

Bevorzugt ist diese Elektrodenleitung eine dauerhaft implantierbare Schraubelektrode mit einer festen oder zurückziehbaren Schraubfixierung am distalen Ende, wobei die Schraubfixierung vorzugsweise einen der elektrischen Pole bildet. Besonders bevorzugt wird die Schraubfixierung in einer Schraubenführung geführt, welche sich am distalen Ende befindet und die distale Öffnung radial zumindest teilweise umgibt. Dadurch wird die Elektrodenleitung auch unter schwierigsten Platzbedingungen leicht implantierbar.

Gemäß einer weiteren Alternative ist das medizinische Implantat als Führungskatheter ausgelegt und umfasst neben dem Hohlraum zur Führung der erfindungsgemäßen Vorrichtung einen zweiten durchgängig hohlen Hohlraum entlang ein- und derselben Längsachse mit einem distalen und einem proximalen Ende, wobei der Hohlraum so beschaffen ist, dass er ein medizinisches Gerät aufnehmen und führen und/oder haltern kann. Der Hohlraum bildet jeweils eine weitere Öffnung am proximalen und am distalen Ende aus, durch die das medizinische Gerät dringen kann. Weiterhin umfasst der Führungskatheter eine Arretierungseinheit am proximalen Ende, welche eine lösbare Arretierung mittel- oder unmittelbar mit der Vorrichtung ermöglicht, um die Lage zwischen Vorrichtung und Führung einzustellen oder eine Verschiebung zwischen Vorrichtung und Führung zu verhindern. Der zweite Hohlraum ist geeignet, um beispielsweise eine Elektrodenleitung zielgerichtet platzieren zu können. Es ist auch vorstellbar, eine gezielte Einführung anderer therapeutischer Implantate zu ermöglichen, beispielsweise Medikamentenkatheter oder Katheter, mit deren Hilfe Gewebe entnommen werden kann oder um beispielsweise Körperlumen offen zu halten oder zu eröffnen. Weiterhin kann ein solcher Katheter auch vorgesehen sein, um beispielsweise Sensoren oder Medikamentendepots zielgerichtet platzieren zu können.

Um den Führungskatheter auch zur Implantation von Elektrodenleitungen einsetzen zu können, welche eine relativ große Anschlusseinrichtung zum Anschluss an ein implantierbares oder externes medizinisches Gerät aufweisen, ist der Hohlkörper peel- oder schlitzbar, so dass die Seitenwand des Hohlraums enlang der Längsachse geöffnet wird. Dadurch ist es möglich, nach der erfolgreichen Fixierung des Implantats den zweiten Hohlraum komplett entlang der Längsachse zu eröffnen, um den Katheter dann nach Freigabe der Fixiereinrichtung der erfindungsgemäßen Vorrichtung komplett zu entnehmen, wobei das Implantat im Körper verbleibt.

Weitere Ziele, Merkmale, Vorteile und Anwendungsmöglichkeiten der Erfindung ergeben sich aus der nachfolgenden Beschreibung von Ausführungsbeispielen anhand der Figuren. Dabei bilden alle beschriebenen und/oder bildlich dargestellten Merkmale für sich oder in beliebiger Kombination den Gegenstand der vorliegenden Erfindung, unabhängig von ihrer Zusammenfassung in den einzelnen Ansprüchen oder deren Rückbeziehungen. Es zeigen:
- Fig. 1a, Fig. 1b: Prinzipielle Darstellung der temporären Befestigung mit einer Vor- richtung nach Anspruch 1
- Fig. 2: das distale Ende eines Ausführungsbeispiels der Vorrichtung nach Anspruch 1 mit einer aktiv fixierbaren Elektrodenleitung
- Fig. 3a, Fig. 3b: ein zweites Ausführungsbeispiel der Vorrichtung mit einer aktiv fi- xierbaren Elektrodenleitung und einer Mandrinhülse
- Fig. 4: das distale Ende eines dritten Ausführungsbeispiels der Vorrichtung mit einem Führungskatheter

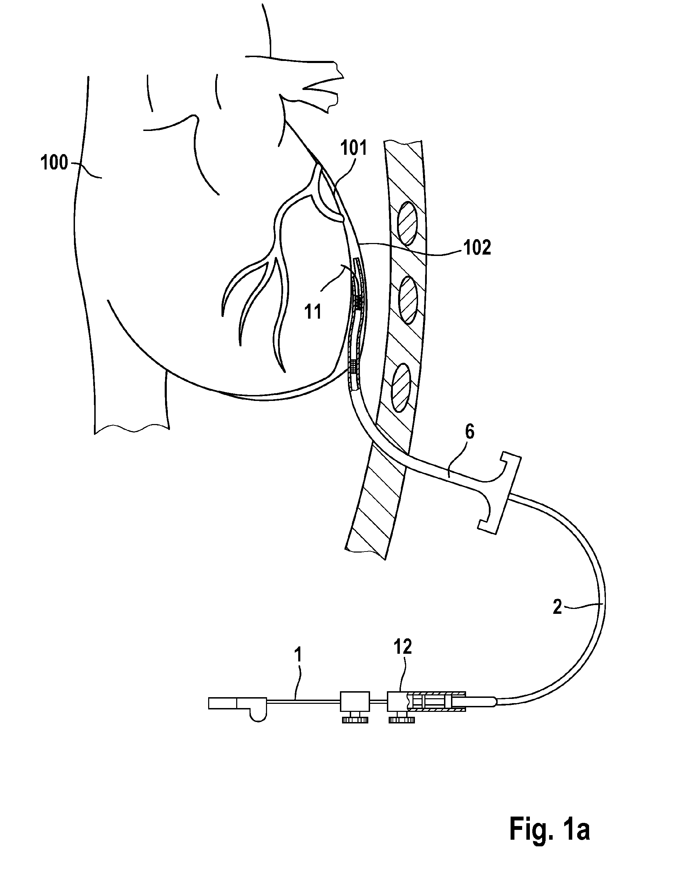

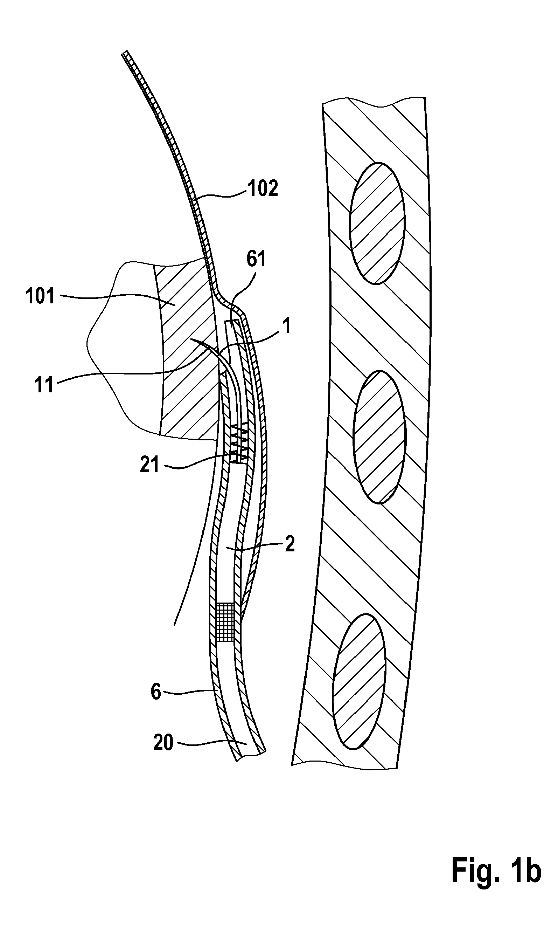

Fig. 1 zeigt eine prinzipielle Darstellung des Einsatzes der erfindungsgemäßen Vorrichtung 1 bei der zielgerichteten Platzierung einer Schraubelektrodenleitung 2 auf dem Epikard 101 eines Herzens 100. Mit Hilfe eines Sheets 6 wird der Brustkorb eröffnet und der distale Bereich 61 des lang gestreckten Hohlkörpers 60 des Sheets 6 bis in zum Perikard 102 vorgeschoben, welches dann vom Sheet 6 durchstoßen wird, so dass das Epikard 101 erreicht wird. Das Sheet 6 hat am distalen Ende einen in laterale Richtung offenen Bereich, welcher direkt am Epikardgewebe zu liegen kommt.

In Fig. 1a wird die Problematik deutlich, welche mit der Vorrichtung behoben werden soll. Denn es ist im Perikard 102 nur möglich, tangential das Epikardgewebe zu erreichen, ohne beispielsweise eine versehentliche Punktion des Herzens herbeizuführen, was zu lebensbedrohlichen Zuständen führen kann. In dieser tangentialen Herangehensweise ist es nicht möglich, eine Schraubelektrodeleitung 2 zu befestigen, da die Schraubfixierung keine Möglichkeit hat, in das Epikardgewebe einzudringen.

Nachdem der Sheet 6 an der gewünschten Stelle liegt, wird eine Vorrichtung 1 mit eine Fixierungseinrichtung 11 durch den Hohlraum 22 des Hohlkörpers 20 in die Elektrodenleitung 2 geschoben. Die Vorrichtung 1 wird dann mit Hilfe der Arretierung 12 so fixiert, dass die Fixierungseinrichtung 11 so im Hohlraum 22 zum liegen kommt, dass sie nicht lateral aus der Elektrodenleitung heraussteht. In diesem Zustand werden die befestigte Vorrichtung 1 und die Elektrodenleitung 2 bis zum distalen Ende 61 des Sheets 6 durch den Hohlkörper 60 vorgeschoben. Ist das distale Ende der Elektrodenleitung am distalen Ende 61 des Sheets 6 angelangt, wird die Elektrodenleitung 2 mit geeigneten Mitteln mit dem Sheet lösbar verbunden, während die Arretierung 12 der Vorrichtung geöffnet wird. Nun kann die vorgebogene und angespitzte Fixierungseinrichtung 11 aus der distalen und lateralen Öffnung des Sheets 6 heraustreten und mit einer definierten Eindringtiefe in das Epikard 101 eindringen und dort befestigt werden. Durch diese Befestigung und die Vorbiegung der Fixierungseinrichtung 11 bildet sich ein Bogen aus, der in einem Winkel von 70 bis 90 Grad in das Epikard 101 eindringt. Jetzt kann die Elektrodenleitung 2 vorgeschoben werden und kann bedingt durch den eingestellten Winkel befestigt werden, da die Schraubfixierung 21 der Elektrodenleitung 2 jetzt die Möglichkeit zum Eingriff in das Epikard hat.

Nach Befestigung der Elektrodenleitung 2 wird die Fixierungseinrichtung 11 aus dem Epikard 101 gezogen und die Vorrichtung 1 zusammen mit dem Sheet 6 entfernt.

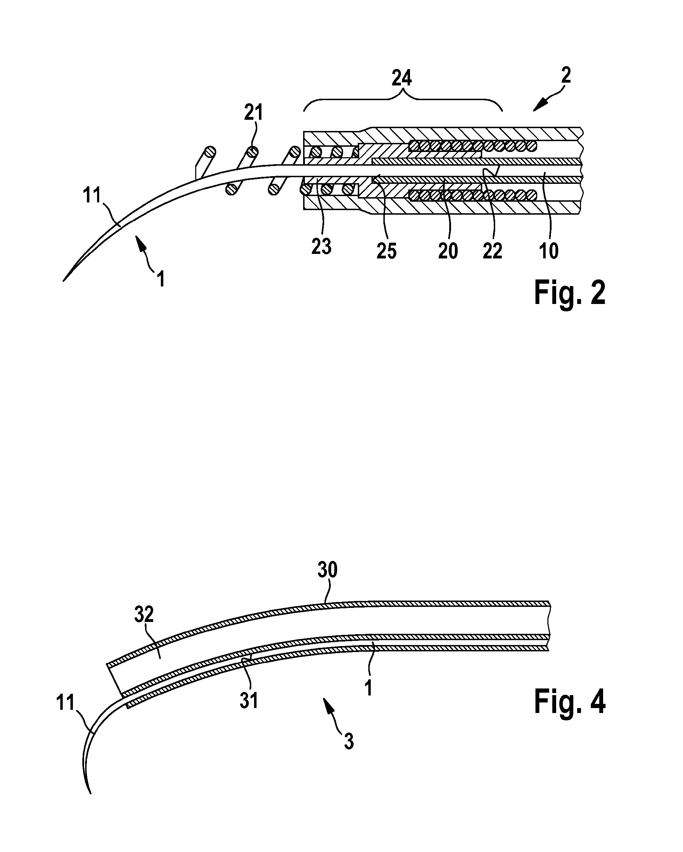

Fig. 2 zeigt eine dauerimplantierbare Elektrodenleitung 2 mit fester oder zurückziehbarer Schraubfixierung 21 und einer Vorrichtung 1 zum zielgerichteten dauerhaften oder vorübergehenden Einbringung von Implantaten.

Die Vorrichtung 1 ist in diesem und den anderen Ausführungen als Draht ausgebildet und weist einen lang gestreckten Körper 10 mit einer Fixierungseinheit 11 auf, die als nadelförmige und vorgebogene, elastische Spitze gebildet ist. Die Fixierungseinheit 11 weist einen Radius von min 5mm bis max. 20mm auf und ist in einem Kreisbogen mit einem Winkel von 70 bis 90 Grad gebogen. Die Orientierung des Bogens ist vom Anwender frei wählbar/einstellbar ist. Im Durchmesser weist der Draht 1 eine übliche Größe auf, die circa. 0,36 mm beträgt. An der Vorrichtung 1 kann eine Markierung vorgesehen sein, mit Hilfe derer festgestellt werden kann, in wie weit die Fixiereinheit 11 ausgefahren wurde.

Die Elektrodenleitung weist mindestens einen Pol, der auch von der Schraubfixierung gebildet werden kann, und einen Hohlkörper 20 auf, der einen durchgehenden Hohlraum 22 mit einer distalen und einer proximalen Öffnung aufweist. Der Hohlraum 22 ist so ausgelegt, dass eine Vorrichtung 1 darin geführt werden kann.

Weiter weist die Elektrodenleitung 2 am distalen Ende eine Dichteinheit 23 auf, die verhindert, dass Blut eintreten kann und die nur von der Fixierungseinheit 11 der Vorrichtung reversibel durchstoßen werden kann. So kann der Draht 1 am distalen Ende der Elektrodenleitung 2 austreten und vorübergehend mit dem Gewebe des Lebewesens verbunden werden.

Die Schraubfixierung 21 ist in einem starren Gehäuse 24 am distalen Ende der Elektrodenleitung 2 gleitend befestigt, so dass sie eine kombinierte Dreh-/Gleitbewegung zum Ein- oder Ausschrauben aus dem Körpergewebe ausführen kann. Durch eine Drehbewegung, die am proximalen Ende der Elektrodenleitung durchgeführt wird und über ein Übertragungselement auf die Schraubfixierung 21 wirkt, kann diese ein- oder ausgeschraubt werden. Das Gehäuse 24 hat dabei eine Ausdehnung entlang der Längsachse der Elektrodenleitung 2 von kleiner 15mm.

An seinem proximalen Ende weist die Elektrodenleitung eine Steckverbindung auf, mit der einer hier nicht dargestellten Anschlusseinheit. Mit dieser Anschlusseinheit, meist in Form eines normierten Steckers gemäß Standard IS-1, DF-1 und/oder IS-4, kann eine elektrische Verbindung mit einem implantierbaren oder externen elektrischen Gerät hergestellt werden. Dabei handelt es sich im Herzschrittmacher, Cardioverter/Defibrillatoren, Hirn-oder Nervenschrittmacher oder andere elektrisch aktive Geräte.

An diesem proximalen Ende der Elektrodenleitung 2 kann der Draht 1 mittels einer an ihm befindlichen Arretierung 12 lösbar verbunden werden, um zu verhindern, dass die Spitze ungewollt am distalen Ende der Elektrode austritt. Die Arretierung kann durch Schrauben, Rasten oder Klemmen erfolgen. Weiterhin kann am proximalen Ende der Elektrodenleitung 2 oder in dessen Nähe eine von außen sichtbare Markierung vorgesehen sein, mit der festgestellt werden kann, ob die Elektrodenleitung durch den Einführkatheter bis zur Herzwand vorgedrungen ist.

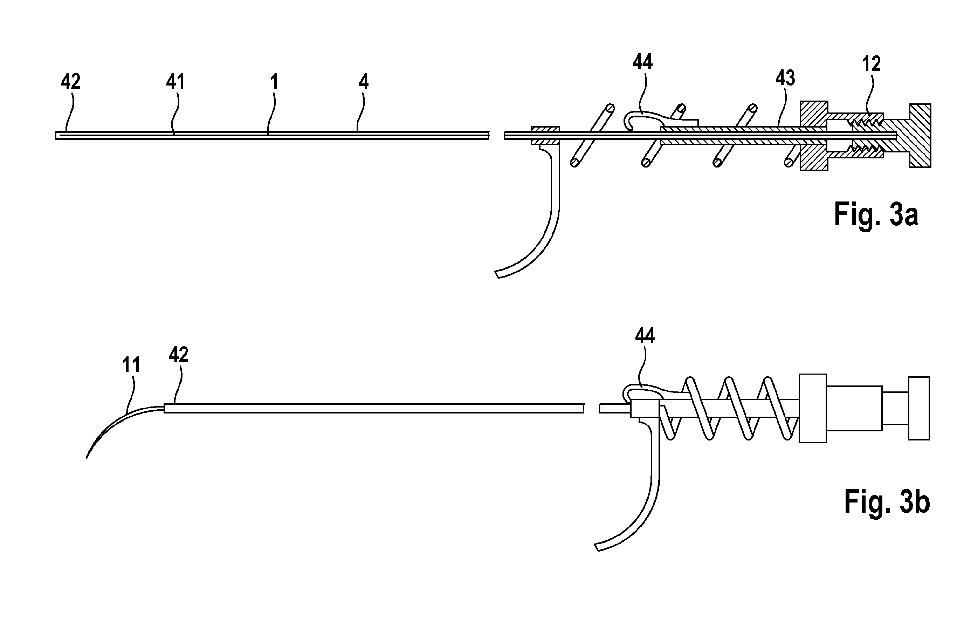

Fig. 3a und 3b zeigen eine weitere Ausführung der Vorrichtung 1 zum zielgerichteten dauerhaften oder vorübergehenden Einbringung von Implantaten.

Die Vorrichtung 1 in Form eines Drahtes verläuft dabei verschiebbar in einer Führung 4 in Form einer Mandrinführungshülse. Eine solche Führung 4 wird dazu verwendet, die Elektrodenleitung 2 leichter an den Implantationsort zu führen und weist einen Hohlkörper 40 mit einem durchgängigen Hohlraum 41 und einem distalen und einem proximalen Ende 42 und 43 auf.

Damit die Elektrodenleitung geführt werden kann, weist die Elektrodenleitung 2 zusätzlich am distalen Ende unmittelbar proximal der Dichteinheit 23 einen üblichen Anschlag 25 auf, der eine Bohrung und ein Widerlager umfasst. Ein solcher Anschlag ist beispielsweise aus der EP 1 356 845 A1 oder der EP 1 452 200 A1 bekannt. Die Bohrung ist dabei so ausgestaltet, dass die Fixierungseinheit 11 des Drahtes 1 durchdringen kann, während die Führung 4 am Widerlager anschlägt, wodurch möglich wird, dass die Elektrodenleitung mit Hilfe der Führung 4 geführt werden kann. Die Führung hat einen Innendurchmesser, der es erlaubt, einen Draht mit Durchmesser 0,36 mm verschiebbar zu führen, also einen Innendurchmesser, der etwas größer als 0,36 mm ist, vorzugsweise 0,4 mm. Weiter weist die Führung 4 an seinem proximalen Ende eine Arretierung 44 auf, mit Hilfe derer der Draht 1 mit der Führung 4 lösbar verbunden werden kann, um zu verhindern, dass die Spitze ungewollt am distalen Ende 42 Führung 4 austritt.

Auch der Draht 1 dieses Ausführungsbeispiels kann an seinem proximalen Ende mittels einer am Draht 1 befindlichen Arretierung 12 lösbar verbunden werden, um eine Verschiebung des vorübergehenden Verbundes aus Draht 1 und Führung 4 relativ zur Elektrodenleitung 3 zu verhindern. Auch hier können die Arretierungen 12 und 44 durch Schrauben, Rasten oder Klemmen erfolgen. Nach dem Lösen der Arretierung 45 kann die Fixierungseinheit 11 des Drahtes 1 in definierter Länge durch den Anschlag 25 und die Dichtung 23 aus dem distalen Ende der Elektrodenleitung ausfahren und im Gewebe des Lebewesens eindringen.

Fig. 4 zeigt ein weiteres Ausführungsbeispiel, bestehend aus einem Führungskatheter 3 als medizinisches Implantat und einer Vorrichtung 1.

Der Führungskatheter 3 weist einen Doppel-Hohlkörper 30 mit zwei parallel verlaufenden Hohlräumen 31 und 32 auf, wobei der Hohlkörper 31 ausgestaltet ist, den Draht 1 verschiebbar zu führen. Der weitere Hohlraum 32 ist ausgestaltet, dass der nicht in Verbindung mit dem Hohlraum 31 steht. Weiter ist der Hohlraum 32 so gestaltet, dass er beispielsweise eine aktiv fixierbare Elektrodenleitung führen kann. Hohlraum 32 ist weiterhin peel- und/oder schlitzbar gestaltet. Beide Hohlräume sind durchgehend hohl und bilden jeweils am distalen und proximalen Ende des Katheters 3 eine Öffnung aus.

An seinem proximalen Ende weist der Führungskatheter 3 eine Arretierung 33 auf, die den Draht 1 so arretiert, dass er nicht aus der Katheterspitze ungewollt austreten kann, aber nach lösen der Arretierung in einer einstellbaren Länge ausgefahren werden kann.

## Patentansprüche

1. Vorrichtung (1) zum zielgerichteten dauerhaften oder vorübergehenden Einbringen von medizinischen Implantaten (2, 3) in ein Lebewesen, welche einen lang gestreckten Körper mit einem distalen Ende und einem proximalen Ende umfasst, wobei die Vorrichtung ausgelegt ist, über einen Zugang in das Lebewesen geführt zu werden, so dass das proximale Ende außerhalb des Lebewesens verbleibt,
**dadurch gekennzeichnet, dass**
das distale Ende eine Fixierungseinrichtung (11) aufweist, mit der die Vorrichtung lösbar mit dem Körpergewebe (101) fest verbunden werden kann.

2. Vorrichtung nach Anspruch 1, **dadurch gekennzeichnet, dass** die Fixierungseinrichtung (11) ausgebildet ist, in das Körpergewebe (101) einzudringen, vorzugsweise angespitzt ist, besonders bevorzugt nadelförmig ist.

3. Vorrichtung nach einem der Ansprüche 1 oder 2, **dadurch gekennzeichnet, dass** die Fixierungseinrichtung (11) eine in laterale Richtung erstreckende Vorbiegung aufweist, welche vorzugsweise einen Radius von 5 mm bis 20 mm und/oder einen Kreisbogen mit einem Winkel von 70° bis 90° im Winkelmaß aufweist.

4. Vorrichtung nach einem der Ansprüche 1 bis 3, **dadurch gekennzeichnet, dass** der Körper und die Fixierungseinrichtung (11) elastisch sind, vorzugsweise bestehend aus einem Draht, besonders bevorzugt einem Draht mit einem Durchmesser von kleiner gleich 0,36 mm.

5. Vorrichtung nach einem der Ansprüche 1 bis 4, **dadurch gekennzeichnet, dass** das proximale Ende eine Arretierungsvorrichtung (12) aufweist, welche eine lösbare Arretierung mittel- oder unmittelbar mit dem medizinischen Implantat (2, 3) ermöglicht, um die Lage zwischen Vorrichtung und medizinischen Implantat einzustellen oder eine Verschiebung zwischen Vorrichtung und Implantat zu verhindern.

6. Vorrichtung nach einem der Ansprüche 1 bis 5, **dadurch gekennzeichnet, dass** die Vorrichtung (1) weiter umfasst:
- eine Führung (4) zur reversiblen Führung oder Halterung der Vorrichtung mit einem lang gestreckten Hohlkörper (40) mit einem distalen und einem proximalen Ende (42, 43) und einem durchgängigen Hohlraum (41) entlang einer Längsachse, wobei der Hohlkörper so beschaffen ist, dass er die Vorrichtung gemäß einem der Ansprüche 1 bis 5 aufnehmen und reversibel führen und/oder haltern kann,
- jeweils einer Öffnung am proximalen und am distalen Ende, welche mit dem Hohlraum (41) des Hohlkörpers verbunden sind, und durch die die Vorrichtung dringen kann, und
- eine Arretierungseinheit (44) am proximalen Ende, welche eine lösbare Arretierung mittel- oder unmittelbar mit der Vorrichtung ermöglicht, um die Lage zwischen Vorrichtung und Führung einzustellen oder eine Verschiebung zwischen Vorrichtung und Führung zu verhindern.

7. Vorrichtung nach Anspruch 6, **dadurch gekennzeichnet, dass** die Führung (4) als Hülse, vorzugsweise als elastische biegbare Mandrinhülse ausgebildet ist.

8. Medizinisches Implantat (2, 3) zum dauerhaften oder vorübergehenden Verbleib in einem Lebewesen, umfassend
- einen Hohlkörper (20, 30) mit einem distalen und einem proximalen Ende und einem durchgängigen Hohlraum (22, 31) entlang einer Längsachse, wobei der Hohlkörper so beschaffen ist, dass er die Vorrichtung (1) gemäß einem der Ansprüche 1 bis 7 aufnehmen und reversibel führen kann, und
- jeweils einer Öffnung am proximalen und am distalen Ende, welche mit dem Hohlraum (22, 31) des Hohlkörpers (20, 30) verbunden sind, durch die die Vorrichtung dringen kann,
**dadurch gekennzeichnet, dass**
die Öffnung am distalen Ende eine von der Fixierungseinrichtung (11) der Vorrichtung reversibel durchstoßbare Dichteinheit (23) zur Abdichtung des Hohlkörpers aufweist.

9. Medizinisches Implantat nach einem der Ansprüche 6 bis 8, **dadurch gekennzeichnet, dass** sich im Hohlkörper (20, 30) unmittelbar proximal vor der Dichteinheit (23) eine Anschlageinrichtung (25) mit einer Bohrung und einem Widerlager befindet, welche so ausgestaltet ist, dass die Fixierungseinrichtung (11) der Vorrichtung (1) die Bohrung durchdringen kann und die Führung (4) am Widerlager anschlägt.

10. Medizinisches Implantat nach Anspruch 8 oder 9, **dadurch gekennzeichnet, dass** das medizinische Implantat eine medizinische Elektrodenleitung (2) ist, welche umfasst:
- am distalen Ende mindestens einen elektrischen Pol zur Abgabe elektrischer Pulse auf das Körpergewebe des Lebewesens,
- mindestens einen mit jeweils einem dieser Pole elektrisch verbundenen Leiter, der sich vom jeweiligen Pol zum proximalen Ende erstreckt, und
- einer Anschlusseinheit am proximalen Ende, welche mit jedem der elektrischen Leiter verbunden ist, um eine elektrische Verbindung mit einem implantierbaren oder externen elektrischen Gerät herzustellen.

11. Medizinisches Implantat nach Anspruch 10, **dadurch gekennzeichnet, dass** die medizinische Elektrodenleitung (2) eine dauerhaft implantierbare Schraubelektrode mit einer festen oder zurückziehbaren Schraubfixierung (21) am distalen Ende ist, wobei die Schraubfixierung vorzugsweise einen der elektrischen Pole bildet.

12. Medizinisches Implantat nach Anspruch 11, **dadurch gekennzeichnet, dass** die Schraubfixierung in einer Schraubenführung (24) geführt wird, welche sich am distalen Ende befindet und die distale Öffnung radial zumindest teilweise umgibtt.

13. Medizinisches Implantat (3) nach Anspruch 8, **dadurch gekennzeichnet, dass** weiter umfasst ist:
- einen zweiten durchgängig hohlen Hohlraum (32) entlang einer Längsachse mit einem distalen und einem proximales Ende, wobei der Hohlraum so beschaffen ist, dass er ein medizinisches Gerät aufnehmen und führen und/oder haltern kann,
- jeweils eine weitere Öffnung am proximalen und am distalen Ende, welche mit dem Hohlraum (32) verbunden sind, durch die das medizinische Gerät dringen kann, und
- eine Arretierungseinheit am proximalen Ende, welche eine lösbare Arretierung mittel- oder unmittelbar mit der Vorrichtung ermöglicht, um die Lage zwischen Vorrichtung und Führung einzustellen oder eine Verschiebung zwischen Vorrichtung und Führung zu verhindern.

14. Medizinisches Implantat nach Anspruch 13, **dadurch gekennzeichnet, dass** der Hohlkörper (30) peel- oder schlitzbar ist, so dass die Seitenwand des Hohlraums (32) entlang der Längsachse geöffnet wird.
